# EUROPEAN PATENT SPECIFICATION

(11) **EP 1 952 795 B1**
(45) Date of publication and mention of the grant of the patent: **16.11.2016**
(21) Application number: 08001515.9
(22) Date of filing: 28.01.2008
(51) Int. Cl.: A61K 8/06, A61K 8/31, A61K 8/37, A61Q 1/14, A61Q 19/10

(54) **Cleansing composition**
Reinigungsmittelzusammensetzung
Composition de nettoyage

(30) Priority: 29.01.2007 JP 2007018542
(43) Date of publication of application: 06.08.2008
(73) Proprietor: KAO CORPORATION, Chuo-ku Tokyo 103-8210 (JP)
(72) Inventor: Murase, Yasuyuki, Tokyo 131-8501 (JP); Tsuda, Hiroko, Tokyo 131-8501 (JP)
(74) Representative: Hoffmann Eitle

(56) References cited:
- EP-A1- 1 702 607
- JP-A- 4 005 213
- JP-A- H06 262 060
- JP-A- 2005 193 134
- US-A1- 2005 180 942

## Description

### FIELD OF THE INVENTION

The present invention relates to a transparent liquid cleansing composition.

### BACKGROUND OF THE INVENTION

Conventional formulations used for the purpose of dissolving a makeup cosmetic with oil for removal include an emulsifying-type formulation such as a cream or an emulsion in which oil is emulsified in water with a nonionic surfactant, an oil gel-type formulation in which oil is retained in a liquid crystal that is a surfactant structure, or the like, and an oil-type formulation in which a nonionic surfactant is added as an emulsifier to dissolve it in oil.

A durable makeup cosmetic such as a waterproof-type mascara has recently come into fashion, and the waterproof-type mascara forms a wax-adhered coating, and therefore the coating cannot be removed unless dissolved by oil. In the emulsifying-type formulation as described above, oil is finely stably dispersed in an aqueous phase, and therefore the intrinsic cleaning power of the oil cannot be exerted and the makeup removing power is not sufficient. There is a problem that although the oil gel-type formulation in which oil is dispersed or solubilized in a surfactant structure has a makeup removing power, the formulation is thick in a gel form and thus it must be repeatedly rubbed many times sufficiently exert the intrinsic makeup removing power of the oil. Although the oil-type formulation has the highest cleaning power because oil directly acts on dirt, there is a problem that the formulation is oily during use and rinsing and an oily feeling remains.

JP-A-4-5213 describes a one-phase type cleansing composition containing two nonionic surfactants having different HLB values, a water-soluble compound having a hydroxyl group, liquid oil and water and having improved spreadability. However, there is a problem that such a cleansing composition is difficult to maintain stability. Further, this cleansing composition forms a transparent gel (gel; liquid crystal structure) in some cases, which causes a problem in makeup removing properties. In addition, since the cleansing composition has a high compounding ratio of oil and the oil itself has an oily feeling, an oily feeling remains on the skin after washing it off.

JP-A-6-293617 describes a cleaning composition containing two kinds of surfactants, oil and a polar organic solvent in combination in a non-aqueous phase having a wide stability temperature range. However, a refreshed feeling of use as a cleansing composition during wash-off cannot be obtained by this composition because it contains no water.

JP-A-2005-193134 describes a one-phase microemulsion composition containing two kinds of nonionic surfactants, oil, a specific aqueous solvent and water, and being stable in a wide temperature range. However, there is a problem that when this microemulsion composition is used as a cleansing composition, it does not have sufficient cleaning power for makeup cosmetics other than silicone-based cosmetics and a feeling of silicone remains even after rinsing.

JP-A-2000-256124 and JP-A-2000-256132 describe cosmetics for makeup removal prepared using a liquid crystal composition (liquid crystal phase and/or isotropic surfactant continuous phase) containing a nonionic surfactant, a water-soluble substance having a hydroxyl group or polar oil, silicone oil and water. Although it is thought that the cosmetics are well compatible with makeup and have a high makeup removing effect, they are not sufficiently satisfiable in terms of removal of durable makeup such as a waterproof-type mascara.

JP-B-3360341 and JP-B-3760516 describe a transparent solubilized composition of an oil component containing a saccharose fatty acid ester with good stability in a certain temperature range, but the temperature range is not sufficiently wide and a refreshed feeling of use cannot be obtained by this composition after rinsing.

As described above, an oil type makeup remover or an oil-gel type makeup remover forming a liquid crystal structure has disadvantages, and among conventionally known cleansing compositions, there has been no cleansing composition satisfying clean wash-off ability without leaving a feeling of residue and sufficient cleaning power for durable makeup such as a waterproof-type mascara.

### SUMMARY OF THE INVENTION

The present invention provides a transparent liquid cleansing composition containing the following components (A), (B), (C), (D), (E) and (F):
(A) 2 to 12% by weight of a nonionic surfactant having an HLB value of 8 or less,
(B) 10 to 30% by weight of a nonionic surfactant having an HLB value of more than 13,
(C) 1 to 10% by weight of a nonionic surfactant having a branched alkyl group or two or more alkyl groups and having an HLB value of more than 8 and 13 or less,
(D) 10 to 40% by weight of oil having a viscosity of 15 mPa·s or less at 30°C,
(E) 10 to 60% by weight of a water-soluble solvent, and
(F) 5 to 50% by weight of water,
wherein a total content of the components (E) and (F) is 40 to 70% by weight.

### DETAILED DESCRIPTION OF THE INVENTION

The present invention relates to a cleansing composition which can be cleanly washed off without leaving a feeling of residue, gives substantially no oily feeling during use, can rapidly raise waterproof mascara and has excellent stability.

The present inventors have found that a cleansing composition which can be cleanly washed off without leaving a feeling of residue no oily feeling, can rapidly raise durable mascara or the like and has excellent stability can be obtained by using specific three kinds of nonionic surfactants, oil, a water-soluble solvent and water in combination.

The cleansing composition of the present invention is a cleansing composition which can be cleanly washed off without leaving a feeling of residue, gives substantially no oily feeling during use, can rapidly raise durable waterproof mascara or the like and has excellent stability.

The nonionic surfactant of the component (A) used in the present invention has an HLB value of 8 or less.

In this case, HLB (Hydrophile-Lipophile Balance) represents the molecular weight of a hydrophilic group portion occupied in the total molecular weight of a surfactant, and the HLB value for a polyoxyethylene-based nonionic surfactant is determined by the following Griffin's equation.

HLB value = E/5
E: weight% of a polyoxyethylene portion included in a surfactant molecule

Specific examples of the nonionic surfactant include polyglycerol fatty acid esters such as diglyceryl oleate and diglyceryl isostearate; polyglycerol alkyl ethers such as diglycerol 2-ethylhexyl ether and isostearyl glyceryl ether; and polyethylene glycol fatty acid esters such as polyethylene glycol (5) monostearate.

Among these, nonionic surfactants having a branched chain or a double bond (alkene) are preferable, and diglyceryl oleate, diglyceryl isostearate and isostearyl glyceryl ether are more preferable.

One or more components (A) can be used and are contained in an amount of 2 to 12% by weight, preferably 2 to 10% by weight in the total composition. It is preferable that the amount of the component (A) is in this range because sufficient cleaning power is realized.

The nonionic surfactant of the component (B) to be used in the present invention is a nonionic surfactant having an HLB value of more than 13.

Specific examples of the nonionic surfactant include polyoxyethylene-based surfactants such as polyoxyethylene fatty acid esters such as polyoxyethylene (12) monolaurate, polyoxyethylene sorbitan fatty acid esters such as polyoxyethylene (20) sorbitan monostearate, polyoxyethylene glycerol fatty acid esters such as polyoxyethylene (20) glycerol monostearate, or the like; saccharose fatty acid esters such as saccharose stearate; and alkyl polyglucosides, and a combination of an alkyl glucoside, a polyoxyethylene-based surfactant or a saccharose fatty acid ester is preferable from a viewpoint of stability and cleaning properties.

Among these, polyoxyethylene fatty acid esters, polyoxyethylene sorbitan fatty acid esters, polyoxyethylene glycerol fatty acid esters and alkyl glucosides having an alkyl group with 8 or more carbon atoms are preferable, and polyoxyethylene fatty acid esters and alkyl glucosides are more preferable.

One or two or more components (B) can be used and are contained in an amount of 10 to 30% by weight, preferably 10 to 25% by weight in the total composition. It is preferable that the amount of the component (B) is in this range because the resulting composition can be cleanly washed off without leaving a feeling of residue.

The nonionic surfactant of the component (C) is a nonionic surfactant having a branched alkyl group or two or more alkyl groups and having an HLB value of more than 8 and 13 or less.

The branched alkyl group preferably has 8 to 18 carbon atoms, and examples thereof include an octyldodecyl group, a hexyldecyl group and an isostearyl group.

Further, the alkyl group preferably has 8 or more carbon atoms.

Specific examples of the nonionic surfactant of the component (C) include polyoxyethylene branched fatty acid esters such as polyoxyethylene (12) monoisostearate; polyoxyethylene sorbitan (di-, tri-, tetra-) fatty acid esters such as polyoxyethylene sorbitan trioleate and polyoxyethylene sorbitan tetraoleate; polyoxyethylene sorbitol (di-, tri-, tetra-)fatty acid esters such as polyoxyethylene sorbitol tetraoleate; polyoxyethylene branched alkyl ethers such as polyoxyethylene (20) octyldodecyl ether; polyoxyethylene hardened castor oil branched fatty acid esters such as polyoxyethylene (60) hardened castor oil monoisolaurate; and polyoxyethylene glycerol branched fatty acid esters such as polyoxyethylene (8) glycerol monoisostearate.

Among these, polyoxyethylene branched fatty acid esters, polyoxyethylene sorbitan (di-, tri-, tetra-)fatty acid esters, polyoxyethylene sorbitol (di-, tri-, tetra-)fatty acid esters, polyoxyethylene branched alkyl ethers and polyoxyethylene glycerol branched fatty acid esters are preferable, and polyoxyethylene sorbitan (di-, tri-, tetra-)fatty acid esters and polyoxyethylene sorbitol (di-, tri-, tetra-)fatty acid esters are more preferable.

One or more components (C) can be used and are contained in an amount of 2 to 5% by weight in total composition. It is preferable that the amount of the component (C) is in this range because the resulting composition can be cleanly washed off without leaving a feeling of residue.

A system containing the components (A), (B) and (C) as nonionic surfactants substantially eliminates the residue feeling of the component (A) and also a plain feeling (non-smooth feeling) of the component (B), so that a smooth rinse feeling can be obtained, when compared to the feeling during rinse with a system containing only the component (A) or (B), or a system containing the components (A) and (B). A weight ratio of the components (A) and (B) to the component (C) is preferably 1:1 to 9:1 ((A) + (B) : (C) = 1:1 to 9:1), more preferably 2:1 to 8:1 because the resulting composition can be cleanly washed off without leaving a feeling of residue.

The oil of the component (D) used in the present invention is liquid at normal temperature and has a viscosity of 15 mPa·s or less, preferably 10 mPa·s or less at 30°C. Here, the viscosity is measured by BM-type viscometer (manufactured by Tokimec Inc., Measurement condition: Rotor No. 1, 60 rpm).

Such oil having a low viscosity has a high permeability to a fine portion and a high solubility of dirt and thus has high cleaning power, so that it has an excellent cleaning power for oily makeup dirt such as oily mascara. Further,a strong oily feeling is not accompanied and the feeling upon use is good.

As such oil, a liquid oil typically used for cosmetics can be used, and there can be used, for example, hydrocarbon oil such as liquid paraffin, liquid isoparaffin, hydrogenated polyisobutene and squalane; ester oil such as cholesteryl isostearate, isopropyl palmitate, isopropyl myristate, neopentyl glycol dicaprate, isopropyl isostearate, octadecyl myristate, cetyl 2-ethylhexanoate, isononyl isononanoate, isotridecyl isononanoate, glycerol tri(2-ethylhexanoate), glycerol tri(caprylate/caprate); ether oil such as alkyl-1,3-dimethylbutyl ether, nonylphenyl ether; methylcyclopolysiloxane such as decamethylcyclopentasiloxane and octamethylcyclotetrasiloxane, silicone oil such as methylpolysiloxane and methylphenylpolysiloxane; animal and plant oils such as olive oil; and terpene oil.

Among these, oil having a molecular weight of 300 or less is further preferable due to its high cleaning power. Specific examples of such oil include isoparaffins such as light liquid isoparaffin and hydrogenated polyisobutene; esters having a branched chain such as isopropyl myristate, isopropyl palmitate and isononyl isononanoate; and cyclic silicones such as octamethyltrisiloxane and octamethylcyclotetrasiloxane. Isoparaffin is more preferable, and isododecane is further more preferable.

Further, isoparaffin and further isododecane are preferably contained in an amount of 30 to 100% by weight in the oil of the component (D) because cleaning power is increased.

Light liquid isoparaffin is generally a mixture of a branched hydrocarbon having 8 to 18 carbon atoms and if a hydrocarbon having a low molecular weight is contained, the isoparaffin has a peculiar smell. Since such a smell is not favored by a user in some cases, preferably, a component of a low molecular weight having 8 to 9 carbon atoms is not contained as much as possible. On the other hand, if the content of a hydrocarbon having 16 to 18 carbon atoms is higher, its cleaning power is poor and an oily feeling left after use tends to be strong. From such a viewpoint, it is preferable to contain a large amount of an isoparaffin-based hydrocarbon having 10 to 15 carbon atoms. Among these, it is preferable to contain a large amount of isododecane having 12 carbon atoms in terms of a balance between smell, use feeling, and cleansing performance.

Examples of the light liquid isoparaffin include those having trade names of Marukasol R (Maruzen Petrochemical Co., Ltd.), IP Solvent 1620, 2028 (both, Idemitsu Petrochemical Co., Ltd.), Isopar L and Isopar H (both, Exxon Chemical Corporation), and Isosol 300, 400 (both, Nippon Petrochemicals Co., Ltd.), and Marukasol R is preferably used from a viewpoint of containing isododecane at a high purity.

One or more components (D) can be used. In addition, oil having a viscosity of more than 15 mPa·s at 30°C may be used. In this case, the oil composition mixed with the oil of the component (B) preferably has a viscosity of 15 mPa·s or less at 30°C.

The oil of the component (D) is contained in an amount of 10 to 40% by weight, preferably 15 to 30% by weight in the total composition. It is preferable that the amount of the oil is in this range because the resulting composition is cleanly washed off without leaving a feeling of residue while maintaining sufficient cleaning power.

The water-soluble solvent of the component (E) is not limited as long as it is those typically used for cosmetics, and examples thereof include monohydric alcohols such as ethanol, propanol, isopropanol, butanol and isobutanol; glycols such as ethylene glycol, propylene glycol, diethylene glycol, dipropylene glycol, 1,3-butylene glycol, 1,4-butylene glycol, hexylene glycol and isoprene glycol; glycerols such as glycerol and diglycerol; saccharides such as sorbitol, multitol, maltose, fructose, xylitol, multotriose, threitol, erithritol and glucose; and saccharide derivatives such as methylglucoside and ethylglucoside. Here, a saccharide which is solid at normal temperature forms an aqueous solution with water of the component (F) described later and the aqueous solution can function as a water-soluble solvent.

Among these, dipropylene glycol, 1,3-butylene glycol, isoprene glycol, glycerol, diglycerol, sorbitol, multitol and the like are preferable.

One or more components (E) can be used and are contained in an amount of 15 to 45% by weight in the total composition. It is preferable that the amount of the component (E) is in this range because sufficient cleaning power and stability are realized.

Water as component (F) is contained in an amount of 5 to 50% by weight, preferably 10 to 40% by weight in the total composition. It is preferable that the amount of the component (F) is in this range because the resulting composition is cleanly washed off without leaving a feeling of residue while maintaining sufficient cleaning power.

The total content of the components (E) and (F) is 40 to 70% by weight, and preferably 50 to 65% by weight. It is preferable that the total content is in this range because feeling upon use without an oily feeling and wash-off properties can be obtained.

The cleansing composition of the present invention preferably has an isotropic liquid phase of a bicontinuous structure formed from the components (A), (B), (C), (D), (E) and (F). The isotropic liquid phase of a bicontinuous structure is an optically isotropic transparent or semitransparent solution having a low viscosity in which both water and oil are continuous. Specifically, the isotropic liquid phase of a bicontinuous structure indicates a bicontinuous microemulsion phase (or µE phase), a middle phase (or D phase) or a sponge phase (or L3 phase).

The cleansing composition of the present invention can exhibit a cleaning function, which has never been sufficiently achieved, by having an isotropic liquid phase of a bicontinuous structure. The structure is one in which both water and oil are continuous, and is excellent in cleaning property for cosmetics, preferably water-resistant mascara and the like. A cosmetic currently used has improved perspiration resistance and sebum resistance, and therefore, it becomes difficult for a conventional cleansing composition to easily wash off the cosmetic. However, a cleansing composition has characteristics that not only affinity for a cosmetic or mascara is excellent, but a wash-off process is greatly facilitated because the composition is very compatible with water even in a wash-off process using water due to an isotropic liquid phase of a bicontinuous structure.

A cleaning agent forming a liquid crystal structure in a system of a nonionic surfactant, oil, a water-soluble solvent and water has been developed so far. However, the present inventors have succeeded in that a bicontinuous structure can be formed in a cleansing composition obtained by using a nonionic surfactant (C) having HLB value(of more than 8 and 13 or less) being in the middle of those of the components (A) and (B) and having a branched alkyl group or two or more alkyl groups even when alignment of a surfactant is disturbed, or an interface between a surfactant and an aqueous phase is disturbed, or the cleansing composition is diluted by dirt such as oil, a little water or the like. Further, they have found that the structure can be remarkably formed by using a branched-type nonionic surfactant as the component (A).

The isotropic liquid phase exhibiting a bicontinuous structure of the cleansing composition according to the present invention can be confirmed by the observation of appearance, observation by an optical polarizing microscope, drawing of a phase diagram, measurement of a self diffusion coefficient by NMR, measurement of electrical conductivity, fluorescent probe method using a fluorescent pigment, or observation by an electronic microscope (such as TEM) in accordance with freeze fracture replica method.

It is possible to distinguish a solution having an isotropic liquid phase exhibiting a bicontinuous structure from the other solution by the observation of appearance, because the former one is in the low-viscosity solution form with a transparent appearance. It is also possible to confirm the isotropic form by arranging two polarizing plates with their polarizing directions perpendicular to each other, and placing, between them, a sample charged in a transparent container. When no light transmission is observed, the solution is confirmed to be isotropic. Through observation by an optical polarizing microscope, the solution can be confirmed to be isotropic if no light transmission is observed at an angle of a polarizing plate set at 90 degree.

Upon confirmation by using a pseudo phase diagram for an aqueous phase (water and water-soluble solvent), an oil phase (oil component) and a surfactant phase, the solution is confirmed to be isotropic if it is in the isotropic liquid form on the phase diagram and is not in a region extending continuously from the apex of the aqueous phase or oil phase. This method is not always applied depending on the substances employed, composition of the aqueous phase or composition of the surfactant phase.

The cleansing composition of the present invention can optionally further contain components typically used for a cleaning agent, for example, thickeners, bactericides, humectants, moisturizers, colorants, antiseptics, feel improvers, perfumes, anti-inflammatory agents, whitening agents, antiperspirants, ultraviolet absorbers, antioxidants, various extract liquids and the like.

The cleansing composition of the present invention can be obtained by appropriately mixing predetermined components, and can be produced by uniformly mixing all the components irrespective of a mixing sequence after heat-melting a raw material that is solid at normal temperature or dissolving it in the other components.

The cleansing composition of the present invention is preferably a transparent liquid cleansing composition.

"Transparent" refers to a state where turbidity is 500 NTU or less by a turbidimeter (TN-100, manufactured by Eutech Instruments).

Further, "liquid" refers to a state where viscosity is 1000 mPa·s or less at 25°C. In this case, the viscosity is measured by a B-type viscometer (Rotor 2, 30 rpm). The composition of the present invention preferably has a viscosity of 500 mPa·s or less at 25°C.

The following examples further describe and demonstrate embodiments of the present invention. The examples are given solely for the purpose of illustration and are not to be construed as limitations of the present invention.

### Examples

### Examples 1 to 22 and Comparative Examples 1 to 12

Cleansing compositions with compositions shown in Table 1 to Table 3 were produced and evaluated for rinsing properties. The results are shown in Table 1 to Table 3 altogether.

### (Production method)

A cleansing composition was obtained by placing all the components (A) to (F) in a container, and stirring and uniformly mixing all the components.

When a component that is solid at room temperature was present, or when a gel component was formed by mixing at room temperature, the solid component or the gel-forming component was heated at 70 to 75°C while stirring to dissolve it. After thoroughly dissolving the component, the temperature was returned to room temperature to obtain a cleansing composition.

All the cleansing compositions of Examples 1 to 22 were transparent liquid cleansing compositions. Further, these cleansing compositions were confirmed to have an isotropic liquid phase of bicontinuous structure by visual observation and observation by an optical polarizing microscope.

### (Evaluation method)

### (1) Rinsing properties:

After about 2 g of each of the cleansing cosmetics (cleansing compositions) was applied to a forearm portion, washed off with water and rinsed, ease of wash-off was evaluated as compared with that obtained by commercially available oil-type makeup removers in accordance with the following criteria.
A; A cleansing cosmetic is very easily washed off as a makeup remover.
B; A cleansing cosmetic is washed off more easily than an oil-type makeup remover.
BC; A cleansing cosmetic is washed off as easily as an oil-type makeup remover.
C; A cleansing cosmetic is more difficult to be washed off than an oil-type makeup remover.
D; Oil persistently remains on the skin and cannot be washed off.

**[Table 1]**

| Component (weight%) | | | Examples | | | | | | | | | | |
|---|---|---|---|---|---|---|---|---|---|---|---|---|---|
| | | HLB | 1 | 2 | 3 | 4 | 5 | 6 | 7 | 8 | 9 | 10 | 11 |
| A | Diglyceryl monoisostearate^{*1} | 8 | 5.0 | | | 5.0 | 5.0 | 5.0 | 5.0 | 4.5 | 5.0 | 2.0 | 10.0 |
| | Diglyceryl monooleate^{*2} | 7 | | 5.0 | | | | | | | | | |
| | Isostearyl glyceryl ether^{*3} | 5 | | | 3.0 | | | | | | | | |
| B | Polyethylene glycol monolaurate^{*4} | 14 | 11.0 | 11.0 | 11.0 | 11.0 | 11.0 | 11.0 | 11.0 | 14.0 | 11.0 | 11.0 | 14.0 |
| | Alkyl (C8 to C16) glucoside^{*5} (40% pure aqueous solution) | 17 | 7.5 | 7.5 | 7.5 | 7.5 | 7.5 | 7.5 | 7.5 | 7.5 | 7.5 | 7.5 | 9.0 |
| C | Polyoxyethylene sorbitol tetraoleate^{*6} | 11 | 3.0 | 3.0 | 3.0 | | | | 2.0 | 3.0 | 5.0 | 3.0 | 4.0 |
| | Polyoxyethylene sorbitan trioleate^{*7} | 11 | | | | 3.0 | | | | | | | |
| | Polyoxyethylene octyldodecyl ether^{*8} | 13 | | | | | 3.0 | | 1.0 | | | | |
| | Polyoxyethylene glycerol branched fatty acid ester^{*9} | 9 | | | | | | 3.0 | | | | | |
| | Triglycerol monostearate^{*10} | 9 | | | | | | | | | | | |
| | Polyoxyethylene sorbitan monostearate^{*11} | 10 | | | | | | | | | | | |
| D | Isododecane (viscosity: 5 mPa·s) | | 6.0 | 6.0 | 6.0 | 6.0 | 6.0 | 6.0 | 6.0 | 6.0 | 6.0 | 6.0 | 6.0 |
| | Isopropyl myristate (viscosity: 10 mPa·s) | | 8.0 | 8.0 | 8.0 | 8.0 | 8.0 | 8.0 | 8.0 | 8.0 | 8.0 | 4.0 | 8.0 |
| | Hydrogenated polyisobutene (viscosity: 16.5 mPa·s)^{*12} | | 2.0 | 2.0 | 2.0 | 2.0 | 2.0 | 2.0 | 2.0 | 2.0 | 2.0 | | 2.0 |
| E | Sorbitol | | 15.0 | 15.0 | 15.0 | 15.0 | 15.0 | 15.0 | 15.0 | 15.0 | 15.0 | 15.0 | 15.0 |
| | 1,3-butylene glycol | | 20.0 | 20.0 | 20.0 | 20.0 | 20.0 | 20.0 | 20.0 | 20.0 | 20.0 | 20.0 | 20.0 |
| | Glycerol | | | | | | | | | | | | |
| | Perfume | | 0.2 | 0.2 | 0.2 | 0.2 | 0.2 | 0.2 | 0.2 | 0.2 | 0.2 | 0.2 | |
| F | Water | | Balance | Balance | Balance | Balance | Balance | Balance | Balance | Balance | Balance | Balance | Balance |
| | Total | | 100 | 100 | 100 | 100 | 100 | 100 | 100 | 100 | 100 | 100 | 100 |
| | Rinsing properties | | A | B | B | A | B | B | B | A | B | A | B |

| | | | | | | | | | | | | | |
|---|---|---|---|---|---|---|---|---|---|---|---|---|---|
| *1: Cosmol 41V (produced by The Nisshin Oillio Group, Ltd.) *2: Poem DO-100V (produced by Riken Vitamin Co., Ltd.) *3: Penetol GE-IS (produced by Kao Corporation) *4: Emanon 1112HG (produced by Kao Corporation) *5: Mydol 10 (produced by Kao Corporation) *6: Reodol 430 (produced by Kao Corporation) *7: Reodol TW-O320V (produced by Kao Corporation) *8: Emulgen 2020G-HA (produced by Kao Corporation) *9: GWIS-108 (produced by Nihon Emulsion Co., Ltd.) *10: Poem TRS-100 (produced by Riken Vitamin Co., Ltd.) *11: Reodol TW-S106 (produced by Kao Corporation) *12: Parleam Ex (produced by NOF Corporation) | | | | | | | | | | | | | |

**[Table 2]**

| Component (weight%) | | | Examples | | | | | | | | | | |
|---|---|---|---|---|---|---|---|---|---|---|---|---|---|
| | | HLB | 12 | 13 | 14 | 15 | 16 | 17 | 18 | 19 | 20 | 21 | 22 |
| A | Diglyceryl monoisostearate^{*1} | 8 | 5.0 | 7.0 | 6.0 | 5.0 | 2.5 | 7.0 | 5.0 | 5.0 | 5.0 | 7.5 | 5.0 |
| | Diglyceryl monooleate^{*2} | 7 | | | | | | | | | | | |
| | Isostearyl glyceryl ether^{*3} | 5 | | | | | | | | | | | |
| B | Polyethylene glycol monolaurate^{*4} | 14 | 7.0 | 25.0 | 11.0 | 16.0 | 5.5 | 21.0 | 11.0 | 11.0 | 11.0 | 16.5 | 14.0 |
| | Alkyl (C8 to C16) glucoside^{*5} (40% pure aqueous solution) | 17 | 7.5 | 7.5 | 7.5 | 7.5 | 3.75 | 7.5 | 7.5 | 7.5 | 7.5 | 11.3 | |
| C | Polyoxyethylene sorbitol tetraoleate^{*6} | 11 | 3.0 | 3.0 | 1.0 | 7.0 | 1.5 | 3.0 | 3.0 | 3.0 | 3.0 | 4.5 | 3.0 |
| | Polyoxyethylene sorbitan trioleate^{*7} | 11 | | | | | | | | | | | |
| | Polyoxyethylene octyldodecyl ether^{*8} | 13 | | | | | | | | | | | |
| | Polyoxyethylene glycerol branched fatty acid ester^{*9} | 9 | | | | | | | | | | | |
| | Triglycerol monostearate^{*10} | 9 | | | | | | | | | | | |
| | Polyoxyethylene sorbitan monostearate^{*11} | 10 | | | | | | | | | | | |
| D | Isododecane (viscosity: 5 mPa·s) | | 6.0 | 6.0 | 6.0 | 6.0 | 4.0 | 8.0 | 8.0 | 10.0 | 10.0 | 17.0 | 8.0 |
| | Isopropyl myristate (viscosity: 10 mPa·s) | | 6.0 | 8.0 | 8.0 | 8.0 | 5.0 | 10.0 | 10.0 | 10.0 | 10.0 | 17.0 | 10.0 |
| | Hydrogenated polyisobutene (viscosity: 16.5 mPa·s)^{*12} | | | 2.0 | 2.0 | 2.0 | 1.0 | 2.0 | 2.0 | | | | 2.0 |
| E | Sorbitol | | 15.0 | 15.0 | 15.0 | 15.0 | 5.0 | 18.0 | 22.0 | | 15.0 | 12.0 | 10.0 |
| | 1,3-butylene glycol | | 20.0 | 20.0 | 20.0 | 20.0 | 10.0 | 18.0 | 22.0 | 15.0 | | 12.0 | 20.0 |
| | Glycerol | | | | | | 5.0 | | | | | | 10.0 |
| | Perfume | | | | | | 0.1 | 0.2 | 0.2 | 0.2 | 0.2 | 0.2 | 0.2 |
| F | Water | | Balance | Balance | Balance | Balance | Balance | Balance | Balance | Balance | Balance | Balance | Balance |
| | Total | | 100 | 100 | 100 | 100 | 100 | 100 | 100 | 100 | 100 | 100 | 100 |
| | Rinsing properties | | B | B | B | A | A | B | A | B | B | B | B |

**[Table 3]**

| Component (weight%) | | | Comparative Example | | | | | | | | | | | |
|---|---|---|---|---|---|---|---|---|---|---|---|---|---|---|
| | | HLB | 1 | 2 | 3 | 4 | 5 | 6 | 7 | 8 | 9 | 10 | 11 | 12 |
| A | Diglyceryl monoisostearate^{*1} | 8 | 7.0 | | 5.0 | 5.0 | 2.0 | 7.0 | 5.0 | 15.0 | 5.0 | 5.0 | 5.0 | 2.5 |
| | Diglyceryl monooleate^{*2} | 7 | | | | | | | | | | | | |
| | Isostearyl glyceryl ether^{*3} | 5 | | | | | | | | | | | | |
| B | Polyethylene glycol monolaurate^{*4} | 14 | 14.0 | 14.0 | 11.0 | 11.0 | 14.0 | 14.0 | 11.0 | 25.0 | 7.0 | 11.0 | 11.0 | 5.5 |
| | Alkyl (C8 to C16) glucoside^{*5} (40% pure aqueous solution) | 17 | 10.0 | 10.0 | 7.5 | 7.5 | 10.0 | 10.0 | 7.5 | 7.5 | | 7.5 | 7.5 | 3.75 |
| C | Polyoxyethylene sorbitol tetraoleate^{*6} | 11 | | 7.0 | | | | | 3.0 | 3.0 | 3.0 | 10.0 | 3.0 | 1.5 |
| | Polyoxyethylene sorbitan trioleate^{*7} | 11 | | | | | | | | | | | | |
| | Polyoxyethylene octyldodecyl ether^{*8} | 13 | | | | | | | | | | | | |
| | Polyoxyethylene glycerol branched fatty acid ester^{*9} | 9 | | | | | | | | | | | | |
| | Triglycerol monostearate^{*10} | 9 | | | 3.0 | | | | | | | | | |
| | Polyoxyethylene sorbitan monostearate^{*11} | 10 | | | | 3.0 | | | | | | | | |
| D | Isododecane (viscosity: 5 mPa·s) | | | | | | 6.0 | 6.0 | 50.0 | 6.0 | 6.0 | 6.0 | 6.0 | 3.0 |
| | Isopropyl myristate (viscosity: 10 mPa·s) | | | | | | 8.0 | 8.0 | | 8.0 | 8.0 | 8.0 | 8.0 | 4.0 |
| | Hydrogenated polyisobutene (viscosity: 16.5 mPa·s)^{*12} | | 15.0 | 15.0 | 15.0 | 15.0 | 2.0 | 2.0 | | 2.0 | 2.0 | 2.0 | 2.0 | 1.0 |
| E | Sorbitol | | 20.0 | 20.0 | 20.0 | 20.0 | 15.0 | 15.0 | 15.0 | 15.0 | 15.0 | 15.0 | 15.0 | 5.0 |
| | 1,3-butylene glycol | | | | | | 20.0 | 20.0 | | 20.0 | 20.0 | 18.0 | 40.0 | 5.0 |
| | Glycerol | | | | | | | | | | | | | |
| | Perfume | | 0.2 | 0.2 | 0.2 | 0.2 | 0.2 | 0.2 | 0.2 | 0.2 | 0.2 | 0.2 | 0.2 | 0.2 |
| F | Water | | Balance | Balance | Balance | Balance | Balance | Balance | Balance | Balance | Balance | Balance | Balance | Balance |
| | Total | | 100 | 100 | 100 | 100 | 100 | 100 | 100 | 100 | 100 | 100 | 100 | 100 |
| | Rinsing properties | | C | D | C | C | C | BC | D | D | C | C | C | BC |

The results in Tables 1-3 showed that all of the cleansing compositions of Examples 1 to 22 could be cleanly washed off without leaving a feeling of residue. Further, these cleansing compositions provided substantially no oily feeling during use and could rapidly raise waterproof mascara and had excellent stability.

## Claims

1. A transparent liquid cleansing composition comprising the following components (A), (B), (C), (D), (E) and (F):
(A) 2 to 12% by weight of a nonionic surfactant having an HLB value of 8 or less,
(B) 10 to 30% by weight of a nonionic surfactant having an HLB value of more than 13,
(C) 2 to 5% by weight of a nonionic surfactant having a branched alkyl group or two or more alkyl groups and having an HLB vlue of more than 8 and 13 or less,
(D) 10 to 40% by weight of oil having a viscosity of 15 mPa·s or less at 30°C,
(E) 15 to 45% by weight of a water-soluble solvent, and
(F) 5 to 50% by weight of water,
wherein the total content of the components (E) and (F) is 40 to 70% by weight of the cleansing composition.

2. The cleansing composition according to claim 1, comprising an isotropic liquid phase of a bicontinuous structure.

3. The cleansing composition according to claim 1 or 2, wherein a weight ratio of the components (A) and (B) to the component (C) is 1:1 to 9:1 ((A) + (B) : (C) = 1:1 to 9:1).

4. The cleansing composition according to any one of claims 1 to 3, wherein the component (A) is a nonionic surfactant having a branched chain or a double bond.

5. The cleansing composition according to any one of claims 1 to 4, wherein the component (A) is at least one or more selected from the group consisting of diglyceryl oleate, diglyceryl isostearate and isostearyl glyceryl ether.

6. The cleansing composition according to any one of claims 1 to 5, wherein the component (B) is at least one or more selected from the group consisting of polyoxyethylene fatty acid esters, polyoxyethylene sorbitan fatty acid esters, polyoxyethylene glycerol fatty acid esters and alkyl glucosides having an alkyl group having 8 or more carbon atoms.

7. The cleansing composition according to any one of claims 1 to 6, wherein the component (C) is at least one or more selected from the group consisting of polyoxyethylene fatty acid esters, polyoxyethylene sorbitan fatty acid esters, polyoxyethylene sorbitol fatty acid esters, polyoxyethylene alkyl ethers and polyoxyethylene glycerol fatty acid esters having a branched alkyl group or two or more alkyl groups.

8. Use of a composition comprising the following components (A), (B), (C), (D), (E) and (F):
(A) 2 to 12% by weight of a nonionic surfactant having an HLB value of 8 or less,
(B) 10 to 30% by weight of a nonionic surfactant having an HLB value of more than 13,
(C) 2 to 5% by weight of a nonionic surfactant having a branched alkyl group or two or more alkyl groups and having an HLB vlue of more than 8 and 13 or less,
(D) 10 to 40% by weight of oil having a viscosity of 15 mPa·s or less at 30°C,
(E) 15 to 45% by weight of a water-soluble solvent, and
(F) 5 to 50% by weight of water,
wherein the total content of the components (E) and (F) is 40 to 70% by weight of the cleansing composition.

## Patentansprüche

1. Transparente flüssige Reinigungszusammensetzung, umfassend die folgenden Komponenten (A), (B), (C), (D), (E) und (F):
(A) 2 bis 12 Gew.% eines nicht-ionischen Tensides mit einem HLB-Wert von 8 oder weniger,
(B) 10 bis 30 Gew.% eines nicht-ionischen Tensides mit einem HLB-Wert von mehr als 13,
(C) 2 bis 5 Gew.% eines nicht-ionischen Tensides mit einer verzweigten Alkylgruppe oder zwei oder mehreren Alkylgruppen und mit einem HLB-Wert von mehr als 8 und 13 oder weniger,
(D) 10 bis 40 Gew.% eines Öls mit einer Viskosität von 15 mPa·s oder weniger bei 30°C,
(E) 15 bis 45 Gew.% eines wasserlöslichen Lösungsmittels und
(F) 5 bis 50 Gew.% Wasser,
wenn der Gesamtgehalt der Komponenten (E) und (F) 40 bis 70 Gew.% der Reinigungszusammensetzung ist.

2. Reinigungszusammensetzung gemäß Anspruch 1, enthaltend eine isotrope Flüssigphase mit einer bikontinuierlichen Struktur.

3. Reinigungszusammensetzung gemäß Anspruch 1 oder 2, worin ein Gewichtsverhältnis der Komponenten (A) und (B) zu der Komponente (C) 1:1 bis 9:1 ((A) + (B) : (C) = 1:1 bis 9:1) ist.

4. Reinigungszusammensetzung gemäß einem der Ansprüche 1 bis 3, worin die Komponente (A) ein nicht-ionisches Tensid mit einer verzweigten Kette oder einer Doppelbindung ist.

5. Reinigungszusammensetzung gemäß einem der Ansprüche 1 bis 4, worin die Komponente (A) zumindest eine oder mehrere ist, ausgewählt aus der Gruppe, bestehend aus Diglyceryloleat, Diglyceryliostearat und Isostearylglycerylether.

6. Reinigungszusammensetzung gemäß einem der Ansprüche 1 bis 5, worin die Komponente (B) zumindest eine oder mehrere ist, ausgewählt aus der Gruppe, bestehend aus Polyoxyethylen-Fettsäureestern, Polyoxyethylensorbitan-Fettsäureestern, Polyoxyethylenglycerin-Fettsäureestern und Alkylglycosiden mit einer Alkylgruppe mit 8 oder mehr Kohlenstoffatomen.

7. Reinigungszusammensetzung gemäß einem der Ansprüche 1 bis 6, worin die Komponente (C) zumindest eine oder mehrere ist, ausgewählt aus der Gruppe, bestehend aus Polyoxyethylen-Fettsäureestern, Polyoxyethylensorbitan-Fettsäureestern, Polyoxyethylensorbit-Fettsäureestern, Polyoxyethylenalkylethern und Polyoxyethylenglycerin-Fettsäureestern mit einer verzweigten Alkylgruppe oder zwei oder mehreren Alkylgruppen.

8. Verwendung einer Zusammensetzung, umfassend die folgenden Komponenten (A), (B), (C), (D), (E) und (F):
(A) 2 bis 12 Gew.% eines nicht-ionischen Tensides mit einem HLB-Wert von 8 oder weniger,
(B) 10 bis 30 Gew.% eines nicht-ionischen Tensides mit einem HLB-Wert von mehr als 13,
(C) 2 bis 5 Gew.% eines nicht-ionischen Tensides mit einer verzweigten Alkylgruppe oder zwei oder mehreren Alkylgruppen und mit einem HLB-Wert von mehr als 8 und 13 oder weniger,
(D) 10 bis 40 Gew.% eines Öls mit einer Viskosität von 15 mPa·s oder weniger bei 30°C,
(E) 15 bis 45 Gew.% eines wasserlöslichen Lösungsmittels und
(F) 5 bis 50 Gew.% Wasser,
wenn der Gesamtgehalt der Komponenten (E) und (F) 40 bis 70 Gew.% der Reinigungszusammensetzung ist.

## Revendications

1. Composition nettoyante liquide, transparente, comprenant les composants qui suivent (A), (B), (C), (D), (E) et (F) :
(A) de 2 à 12 % en poids d'un tensioactif non ionique ayant une valeur HLB de 8 ou moins,
(B) de 10 à 30 % en poids d'un tensioactif non ionique ayant une valeur HLB supérieure à 13,
(C) de 2 à 5 % en poids d'une tensioactif non ionique ayant un groupe alkyle ramifié ou 2 groupes alkyle ou plus et ayant une valeur HLB supérieure à 8 et 13 ou moins,
(D) de 10 à 40 % en poids d'une huile ayant une viscosité de 15 mPa·s ou moins à 30 °C,
(E) de 15 à 45 % en poids d'un solvant hydrosoluble, et
(F) de 5 à 50 % en poids d'eau,
dans laquelle, la teneur totale des composants (E) et (F) est de 40 à 70 % en poids de la composition nettoyante.

2. Composition nettoyante selon la revendication 1, comprenant une phase liquide isotrope d'une structure bicontinue.

3. Composition nettoyante selon la revendication 1 ou 2, dans laquelle un rapport en poids des composants (A) et (B) sur le composant (C) est de 1:1 à 9:1 ((A) + (B) : (C) = de 1:1 à 9:1)).

4. Composition nettoyante selon l'une quelconque des revendications 1 à 3, dans laquelle le composant (A) est un tensioactif non ionique ayant une chaîne ramifiée ou une double liaison.

5. Composition nettoyante selon l'une quelconque des revendications 1 à 4, dans laquelle le composant (A) est au moins l'un ou plus choisis dans le groupe constitué par l'oléate de diglycéryle, l'isostéarate de diglycéryle et l'éther d'isostéaryl glycéryle.

6. Composition nettoyante selon l'une quelconque des revendications 1 à 5, dans laquelle le composant (B) est au moins l'un ou plus choisis dans le groupe constitué par les esters d'acides gras de polyoxyéthylène, les esters d'acides gras de sorbitane de polyoxyéthylène, les esters d'acides gras de glycérol de polyoxyéthylène et les alkylglucosides ayant un groupe alkyle ayant 8 atomes de carbone ou plus.

7. Composition nettoyante selon l'une quelconque des revendications 1 à 6, dans laquelle le composant (C) est au moins l'un ou plus choisis dans le groupe constitué par les esters d'acides gras de polyoxyéthylène, les esters d'acides gras de sorbitane de polyoxyéthylène, les esters d'acides gras de sorbitol de polyoxyéthylène, les éthers alkyliques de polyoxyéthylène et les esters d'acides gras de glycérol de polyoxyéthylène ayant un groupe alkyle ramifié ou deux groupes alkyle ou plus.

8. Utilisation d'une composition comprenant les composants qui suivent (A), (B), (C), (D), (E) et (F) :
(A) de 2 à 12 % en poids d'un tensioactif non ionique ayant une valeur HLB de 8 ou moins,
(B) de 10 à 30 % en poids d'un tensioactif ayant une valeur HLB supérieure à 13,
(C) de 2 à 5 % en poids d'un tensioactif non ionique ayant un groupe alkyle ramifié ou deux groupes alkyle ou plus, et ayant une valeur HLB supérieure à 8 et 13 ou moins,
(D) de 10 à 40 % en poids d'une huile ayant une viscosité de 15 mPa·s ou moins à 30 °C,
(E) de 15 à 45 % en poids d'un solvant hydrosoluble, et
(F) de 5 à 50 % en poids d'eau,
dans laquelle la teneur totale des composants (E) et (F) est de 40 à 70 % en poids de la composition nettoyante.
